# EUROPEAN PATENT SPECIFICATION

(11) **EP 3 484 360 B1**
(45) Date of publication and mention of the grant of the patent: **06.09.2023**
(21) Application number: 17737302.4
(22) Date of filing: 14.07.2017
(51) Int. Cl.: A61B 5/053, A61B 5/145, A61B 5/16, A61B 5/00

(54) **DEVICE FOR ASSESSING PSYCHOPHYSIOLOGICAL RESPONSIVENESS**
VORRICHTUNG ZUR BEURTEILUNG VON PSYCHOPHYSIOLOGISCHER ANSPRECHBARKEIT
DISPOSITIF POUR ÉVALUER LA RÉACTIVITÉ PSYCHOPHYSIOLOGIQUE

(30) Priority: 18.07.2016 EP 16179966
(43) Date of publication of application: 22.05.2019
(73) Proprietor: Koninklijke Philips N.V., 5656 AG Eindhoven (NL)
(72) Inventor: OUWERKERK, Martin, 5656 AE Eindhoven (NL)
(74) Representative: Philips Intellectual Property & Standards
(86) International application number: PCT/EP2017/067899
(87) International publication number: WO 2018/015308

(56) References cited:
- US-A- 3 785 368
- US-A1- 2013 317 318
- US-A1- 2013 338 470
- US-A1- 2016 242 672

## Description

### FIELD OF THE INVENTION

The present invention relates a device to assessing psychophysiological responsiveness of a subject, in particular via a skin conductance measurement.

### BACKGROUND OF THE INVENTION

Skin conductance sensing can be used to detect psychophysiological responses. Emotions can give rise to sweat gland activation via the sympathetic part of the autonomous nervous system. A trigger that invokes an emotional response can cause a peak to appear in the conductance of the skin. Such a peak is commonly called a skin conductance response (SCR). The absence of skin conductance responses thus generally signals the absence of emotionally triggered responses. Hence, a person can be considered to be in a relaxed state during a period without such responses.

US 2014/0155724 A1 discloses an advantageous wearable device for ensuring good electrode-skin contact for reliable skin conductance measurement. In an embodiment, the device comprises at least two skin conductance electrodes for contacting a skin of a user and an elastic material portion surrounding, in particular circumferentially enclosing, the skin conductance electrodes. This enclosing portion is preferably made of an elastic material which is non-permeable for gaseous and liquid substances such that a fluid film can be generated between the skin and the electrodes when the wearable device is worn by the user. This promotes the formation of a microclimate which provides optimum conditions for psychophysiological sweating.

US 2014/0275845 A1 discloses a finger-mounted physiology sensor. The physiology sensor has a sensor body including first and second raised mounds, protruding from its surface and being longitudinally spaced apart such as to define a transverse air gap therein between. The mounds each retain electrodes which abut palmar surfaces of the single finger. The sensor includes a skin conductance sensing device. A processor is housed within the body and communicates with the electrodes and the sensing devices to apply an electrical excitation thereto and to process the physiological measurements. An object of this disclosure is to reduce the risk of skin conductance sweat bridging which occurs when sweat builds up on the surface of the skin between adjacent electrodes such that a current used to excite the electrodes is able to travel directly between the electrodes along the moisture paths created by sweat, and thus not through the skin.

US 2014/0275845 A1 further discloses that a long term application of a DC excitation can lead to electrode polarization, where these electrodes charge up like a battery and oppose the flow of current, thus reducing the apparent skin conductance and skewing skin conductance readings. To overcome this effect, it is suggested to apply a relatively low-frequency excitation using an AC square wave generator, which can apply an AC square wave pulse to the electrodes and the sensing devices. The resulting skin conductance measurement is determined from the difference between the voltages at the ends of AC square wave pulses. The reason for measuring at the ends of AC square wave pulses is that it is known that skin has a significant capacitive component, meaning that after a step in the applied voltage, steady state DC current is not established right away and instead drops off from an initial higher level as the skin capacitance changes. In order to avoid this undesirable effect, US 2014/0275845 A1 teaches to sample the resulting response to the AC excitation after the maximum amount of settling time has elapsed, such as just before the next voltage reversal.

US 2013/0338470 A1 relates to a dry skin conductance electrode for contacting the skin of a user. In order to provide a dry skin conductance electrode for long-term measurements which does not cause problems to the user while still providing a good signal level, the electrode comprises a material made of a noble metal doped with at least one dopant selected from the group consisting of hydrogen, lithium, sodium, potassium, rubidium, caesium and beryllium.

### SUMMARY OF THE INVENTION

It is an object of the present invention to provide a device which support and/or enable skin conductance measurements of improved reliability, in particular when using dry electrodes.

In a first aspect of the present invention a device for assessing a psychophysiological responsiveness of a subject is presented according to claim 1.

In a further aspect of the present invention, a system for assessing a psychophysiological responsiveness of a subject is presented, the system comprising:
- a first electrode and a second electrode for application to a skin of the subject; and
- the aforementioned device for assessing the psychophysiological responsiveness of the subject.

Trustworthy and robust interpretation of skin conductance sensor data can yield important information on a person's mental health and/or stress patterns. The present invention is based on the idea of evaluating the presence or absence of an electrical double-layer in an electrical path between two electrodes for skin conductance measurement to verify whether a subject is in a state of psychophysiological responsiveness or psychophysiological non-responsiveness. Identifying the electrical double-layer, and thereby identifying a state of psychophysiological responsiveness, can thus refer to determining or detecting a presence and/or absence of the electrical double-layer. An electrical double-layer can cause a voltage barrier or blocking voltage which can be evaluated. In addition or in the alternative, an electrical double-layer can be seen as a capacitive contribution that has an impact on a transient behavior of an impedance acquired in response to an electrical stimulus. The presence of transients in the skin conductance data can signal the presence of a salty liquid, i.e. sweat, in the conductive path. In this case the subject can be classified as being in a state of psychophysiological responsiveness. Psychophysiological sweating can thus be evaluated. The transients are caused by the (slow) diffusion of ions in the sweat, responding to the changes in voltage by lowering the space charge close to the electrodes. However, if the transients are absent this effect does not occur, and can be attributed to a conductive path mainly through the stratum corneum. In this case, the person can be classified as being in a state of psychophysiological non-responsiveness. Correspondingly, the presence of a blocking voltage indicates that an electrical double-layer, also referred to as ionic double-layer, has formed, which can serve as an indicator of the presence of a salty liquid comprising ions in the conductive path. In this case the subject can be classified as being in a state of psychophysiological responsiveness.

As indicated above, a period without skin conductance responses (SCRs) can mean a period of relaxation. However, in particular for wearable sensors which use dry electrodes, the absence of SCRs could also be the result of a shortage of sweat between the electrodes and the skin. Such a state however, can be seen as a state of psychophysiological non-responsiveness for a skin conductance sensor. It has been found that also in this case a low but stable conductance signal can measured even though there is no substantial salty liquid connection between sweat gland ducts and the electrode. Hence, a single absolute value of skin conductance is not necessarily a reliable indicator of a reliable measurement. This is particularly true in a non-laboratory environment, for example in the field of wearable consumer or fitness devices such as activity trackers and smart watches. Further, there can be a large spread in absolute skin conductance levels between subjects, for example, due to different skin types and different placement of electrodes.

In particular in cold and/or dry conditions psychophysiological sweating cannot always be detected with a skin conductance sensor. Periods without skin conductance responses can under such conditions erroneously be regarded as periods of relaxation. This is an erroneous conclusion. By determining a state of psychophysiological responsiveness or non-responsiveness of the subject based on the identified electrical double-layer, the present disclosure offers a method to discriminate between a period of true relaxation and a period during which the conditions may prohibit the detection of psychophysiological sweating. Thus, if a period of true relaxation is measured in conjunction with the presence of an electrical double-layer in response to an electrical stimulus - which is indicative of a sweat liquid connection between sweat gland ducts and the electrodes - this can be verified as trustworthy. Further, if a salty liquid connection between sweat gland ducts and the electrode is lacking, measures to establish this connection can be proposed and their effectiveness verified.

An electrical double-layer or ionic double-layer or simply double-layer as used herein can be seen as a structure that appears on the surface of an object when it is exposed to a fluid, for instance exposed to sweat. The double-layer can refer to two substantially parallel layers of charge surrounding the object. If the object is consists of a negatively charged metal, the first layer, the (positive) surface charge, comprises ions adsorbed onto the object due to electrical attraction. The second layer is composed of ions attracted to the surface charge via the coulomb force, electrically screening the first layer. This second layer is loosely associated with the object. It comprises free ions that move in the fluid under the influence of electric attraction and thermal motion rather than being firmly anchored. It is thus called the "diffuse layer".

Further details about the general behavior of transient currents of electrolytes in response to a voltage step can be found in Marescaux et al, "Impact of diffusion layers in strong electrolytes on the transient current", Phys. Rev. E 79, 011502, 2009. This paper by Marescaux illustrates the underlying process wherein transients are caused by an electrolyte responding to changes in voltage by lowering the spaced charge closed to the electrodes. The slow diffusion of ions in sweat can be seen as a transient behavior of an electrolyte. The ions form a blocking layer, which can to a certain extent, be seen similar to a PN junction. However, also due to the physical mass, movements of ions occur on a much slower time scale of for example 300 ms. It should further be noted that the ions forming a double-layer capacitance and blocking voltage also leads to an effective voltage drop or voltage barrier indicative of said double ionic layer that has an impact on a impedance signal acquired in response to an electrical stimulus. Hence, the transient behavior and/or the voltage barrier can be evaluated to determine the presence or absence of an electrical double-layer.

In contrast to US 2014/0275845 A1, wherein the capacitive component of the skin is seen as an undesirable contribution such that a measurement is performed after the maximum amount of settling time has elapsed, the solution according to an aspect of the present invention can make positive use of this effect by evaluating the presence or absence of a transient behavior of the impedance signal. The observed current response after a positive voltage step has been applied is also described in Comunetti et al. "Characterisation of human skin conductance at acupuncture points", Experientia 51, 328-331, 1995. However, no distinction is made between psychophysiological responsiveness and non-responsiveness based on the transient behavior in the prior art.

Literature indicates a base rate of 5-10% non-responders. Several medical or climatic conditions can cause cold hands and cold feet. For example Raynaud's disease causes some areas of your body - such as fingers and toes - to feel numb and cold in response to cold temperatures or stress. Thermal sweating may become inhibited by such conditions, causing psychological sweating to stop too. These subjects can then be in a state of psychophysiological non-responsiveness for skin conductance sensors worn at the wrist or other extremities of the body. Also in this case, the proposed solution can offer benefit in that their state of psychophysiological non-responsiveness is not by mistake classified as a relaxed state.

A sensing unit for acquiring an impedance signal indicative of an impedance between the first and the second electrode can basically be worn on all body locations. Machado-Moreira et al. found that psychological sweating can occur all over the body, as published in "Sudomotor responses from glabrous and non-glabrous skin during cognitive and painful stimulations following passive heating", Acta Physiol, 204, 571-581, 2012. Advantageously, the proposed device for assessing a psychophysiological responsiveness of a subject or at least the sensing unit can be implemented together with a conventional skin conductance sensor or measurement device. Such device can be easily attached to the user, for example as a part of a watch, in particular a smart watch, a separate wristband or a body sensor such as a patch or a belt. Skin conductance measurement can also be referred to as a galvanic skin conductance (GSR) measurement or electrodermal activity (EDA) measurement.

An impedance signal can, for example, refer to an electrical current *I* measured in response to a voltage *U* applied as the electrical stimulus. The impedance can thus be refer to the resistance *R*=*U*/*I.* A corresponding conductance *G* can be determined by the inverse as *G*=*1*/*R*=*I*/*U.* In the given example, a transient behavior of the impedance signal can thus describe how the resistance and/or conductance changes over time. Hence, a transient behavior of the impedance signal can refer to how the impedance changes over time. An impedance signal can also be indicative of a complex impedance, for example indicative of a capacitive contribution for the case of psychophysiological responsiveness.

In an embodiment, the analysis unit can be adapted to discriminate between a state of psychophysiological responsiveness and psychophysiological non-responsiveness, wherein an absence of an electrical double-layer, for example indicated by an absence of a transient behavior in the impedance signal, can be indicative of a state of psychophysiological non-responsiveness of the subject. Correspondingly, the analysis unit can be adapted to discriminate between a state of psychophysiological responsiveness and psychophysiological non-responsiveness, wherein a presence of an electrical double-layer, for example indicated by a presence of a transient behavior in the impedance signal, can be indicative of a state of psychophysiological responsiveness of the subject. Hence, the presence or absence of such a transient behavior and/or voltage barrier can indicate that the subject is in a responsive or non-responsive state. This allows an efficient analysis and determination of a state of psychophysiological responsiveness or non-responsiveness of the subj ect.

In an embodiment, the analysis unit can be configured to identify electrical double-layer by evaluating a voltage barrier indicative of said electrical double-layer based on the impedance signal acquired in response to the electrical stimulus. As indicated above, the ions in the sweat can form a blocking layer which can be seen similar to a PN junction in a semiconductor. For example, different impedances measured in response to different applied currents or voltages can be evaluated to determine the presence or absence of the voltage barrier. For example, a threshold voltage which needs to be applied to overcome the blocking voltage can be determined.

According to the invention, the analysis unit is configured to identify the electrical double-layer based on a transient behavior of said impedance signal. Any suitable algorithm to detect the presence of transient behavior including known algorithms can be used. An exemplary algorithm to detect the presence of transient behavior after voltage changes can use the first derivative of the skin conductance. In the absence of transients the first derivative will be stable and potentially small value not exceeding a predetermined threshold. In the case of transient behavior the first derivative can, for example, be expected to go to a large positive or negative value exceeding the predetermined threshold shortly after a declining or increasing voltage step, and gradually, in particular over a couple of seconds, decline to a lower value. The time scale for a double ionic layer to build up is in the order of 50-500 ms, in particular 300 ms.

The analysis unit is adapted to determine the state of psychophysiological responsiveness of the subject based on a transient behavior of a time scale of 1 ms to 5 s, preferably between 10 ms and 2 s, preferably between 20 ms and 500 ms. An advantage of this embodiment is that neither slow changes like signal drift or a true (slow) psychophysiological skin conductance response (SCR), nor rapid changes which can be attributed for example to artifacts such as repositioning of the electrodes or glitches or peaks from a switching of the stimulus unit, are considered. As indicated above, a typical time to build up the double-layer is 300 ms. The transient behavior of a time scale can refer to a half-value period. It should be understood that the timing may strongly depend on the moisture layer thickness between skin and electrode, which can be variable and may also vary due to motions. Usually, a period of 300 ms can suffice to establish the presence of the transient.

According to the invention, the stimulus unit comprises a voltage source for applying a voltage across the first and the second electrode and/or a current source for applying a current to the first and the second electrode. Hence, a voltage or current stimulus can be applied.

The stimulus unit is adapted to provide an electrical stimulus comprising a step function from a first voltage to a second voltage or correspondingly from a first current to a second current. Hence, the stimulus can advantageously be provided with a fast transition from a first level to a second level. In response to said electrical stimulus, the sensing unit acquires an impedance signal indicative of the impedance between the first and the second electrode. The acquired impedance signal can thus be seen as the step response of the skin of the subject. For the case of a state of psychophysiological non-responsiveness, wherein a transient behavior in the impedance signal is substantially absent, the acquired impedance signal may also show a step-like behavior. However, for the case where the subject is in a state of psychophysiological responsiveness, the impedance signal may not show a step-like behavior but may need some time to settle at a stable value. As explained above, the time scale is predominantly covered by the ion transfer process of ions in the sweat in the skin.

In an embodiment, the stimulus unit can be adapted to provide a voltage and/or current profile. Hence, more generally speaking different voltages in a pre-defined range can be applied to the skin of the subject. For example, a voltage over the two skin electrodes can be altered in a stepwise manner and the impedance signal acquired in response to said voltage profile can be evaluated. A voltage and/or current profile can be advantageous in determining a voltage barrier indicative of the electrical double-layer. It is thus also possible to check the transients corresponding to different voltages. Alternatively or in addition, the applied voltage can simply be reversed or commutated. An advantage of such commutation is the cost effective implementation by means of switches. The concept also applies mutatis mutandis to applying a current profile.

In a refinement, the stimulus unit can be adapted to provide an AC electrical excitation. In other words, an alternating electrical excitation is provided, for example an alternating current or voltage. For example, a sine wave or sinusoidal voltage is provided via the first and the second electrode to the skin of the subject. In this case, the impedance signal indicative of an impedance between the first and the second electrode in response to said alternating electrical stimulus can be a measured current. In this case, a phase difference between the applied AC voltage and the acquired AC current can be evaluated as the transient behavior. Hence, a phase difference between the AC electrical excitation and the measured current can be evaluated. In particular, a phase shift can be evaluated at a frequency close to settling speed or time of the transient behavior, for example at 3 Hz for a settling time of 300 ms. An advantage of this embodiment is that the high frequency (HF) requirements of the electrical components for providing the electrical stimulus and/or for acquiring the impedance signal may be relaxed. Hence, instead of providing a very fast transition from a first voltage to a second voltage, it would be sufficient to supply an alternating voltage with lower edge steepness or slew rate.

In an embodiment, the sensing unit can be adapted to acquire a time-variant current and/or voltage between the first and the second electrode. An exemplary sensing unit may comprise a series resistor connected in series to a lead coming from one of the electrodes, thereby forming a voltage divider. The skin conductance can be determined from the ratio between the total applied voltage and a voltage across the reference resistor or, in the alternative appearing at the electrodes. This configuration can also advantageously be used to determine a voltage barrier formed by an electrical double-layer. The voltage barrier can be seen as a voltage offset that can be detected. The skin impedance, the voltage barrier and the reference resistance can be considered as connected in series. To determine the skin impedance and the voltage barrier, in a first step a first voltage can be applied across the electrodes and a first voltage across the reference resistor can detected. In a second step, a second voltage can be applied across the electrodes and a second voltage across the reference resistor can be detected. Based thereon an equation system can easily be established and solved for the voltage barrier and reference resistance.

In an embodiment, the device further comprises an output unit for providing information indicative of the state of psychophysiological responsiveness or non-responsiveness to the subject, in particular for providing advice how to overcome a state of psychophysiological non-responsiveness. An advantage of this embodiment is that the subject can be aware of whether the device is capable of correctly determining a psychophysiological state, for example a relaxed state. Moreover, the subject can be instructed how to overcome this condition, hence, an advice how to mitigate this condition can be given. For instance, moistening the area under the electrodes with tap water or application of artificial sweat can create a liquid medium in which the transient behavior is expected to re-appear, as well as the psychophysiological responsiveness. Alternative solutions are increasing the ambient temperature, or a short period of intense exercise, such as performing 25 push-ups. Repeating the assessment of psychophysiological responsiveness by again providing an electrical stimulus and evaluating an acquired impedance signal in response to said electrical stimulus can be used to verify the effectiveness of such a measure. It will be appreciated that the effectiveness can also be signaled to the subject.

### BRIEF DESCRIPTION OF THE DRAWINGS

These and other aspects of the invention will be apparent from and elucidated with reference to the embodiments described hereinafter. In the following drawings
Fig. 1 shows a schematic diagram of an embodiment of a system for assessing a psychophysiological responsiveness of a subject;
Fig. 2 shows an exemplary implementation in form of a wearable device such as a smart watch;
Fig. 3 shows a simplified equivalent electrical circuit of skin tissue including sweat glands;
Fig. 4 shows an exemplary flow chart for assessing a psychophysiological responsiveness of a subject; and
Fig. 5A to Fig. 5D show exemplary graphs of provided electrical stimuli and acquired impedance signals in response thereto.

### DETAILED DESCRIPTION OF THE INVENTION

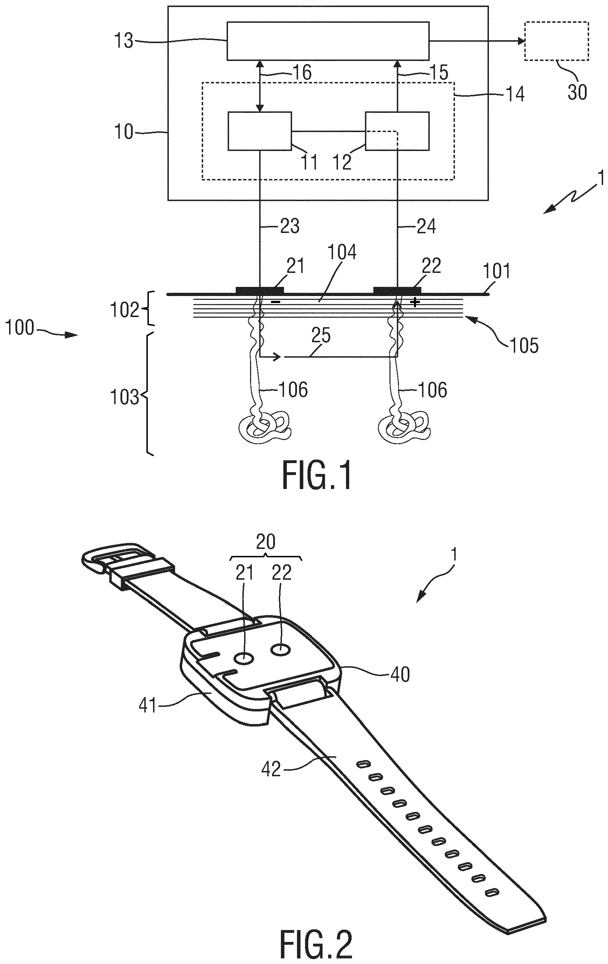

Fig. 1 shows a schematic diagram of a first embodiment of a system 1 for assessing a psychophysiological responsiveness of a subject. The system 1 comprises a device 10 for assessing a psychophysiological responsiveness of the subject as well as a first electrode 21 and a second electrode 22 for application to a skin 100 of the subject.

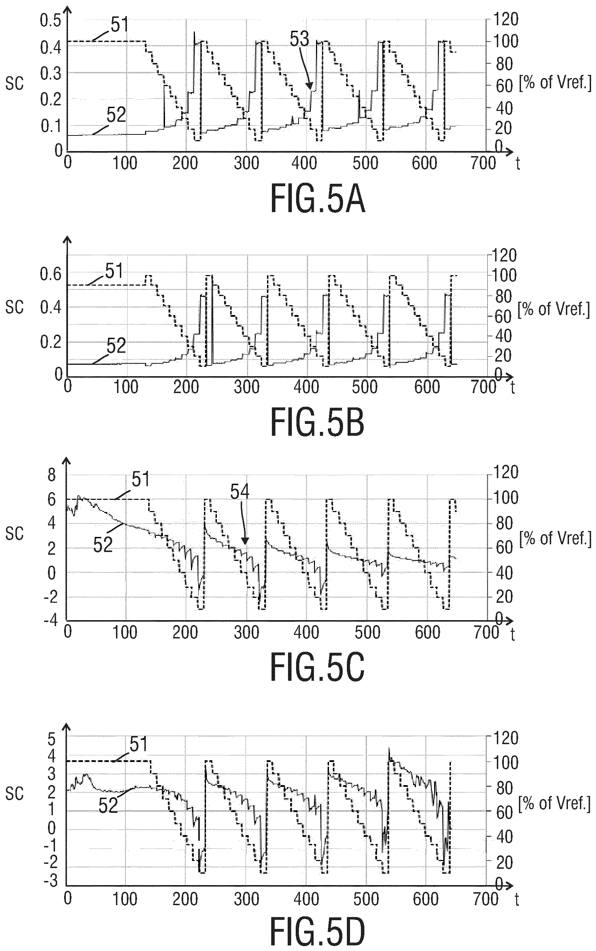

The device 10 comprises a stimulus unit 11 for providing an electrical stimulus via the first electrode 21 and the second electrode 22 to the skin 100 of the subject. In the given example, the stimulus unit comprises a voltage source for applying a voltage across the two electrodes 21, 22. The stimulus unit is connected to the first electrode 21 and the second electrode 22 via electrode leads or cables 23, 24. In particular, the stimulus unit 11 is adapted to provide a voltage profile comprising one or more step functions between different voltage levels as exemplary shown in Fig. 5A Fig. 5D.

The device 10 further comprises a sensing unit 12 for acquiring an impedance signal indicative of an impedance between the first electrode 21 and the second electrode 22. The sensing unit 12 can be a conventional skin conductance sensor as known in the art. The sensing unit can be connected separately to the first electrode 21 and the second electrode 22. However, advantageously, the same electrical connection to the first and the second electrodes 21, 22 via the electrode lead 22, 24 is to be used for both the stimulus unit 11 and the sensing unit 12. In an advantageous embodiment, the sensing unit 12 comprises a series resistor in the electrical path from the stimulus unit to the electrode 22. Hence, this series resistor then forms a voltage divider together with the impedance of the skin 100 of the subject. Thus, a voltage drop across the series resistor or current through the series resistor can be taken as the impedance signal indicative of the impedance of the skin 100 of the subject in response to the electrical stimulus provided by the stimulus unit 11. Hence, the sensing unit 12 does not directly need to measure the conductance or impedance of the skin 100 but determines an impedance signal indicative of the impedance between the first and the second electrode 21, 22 from which the impedance in the first and the second electrode can be derived using known principles.

In an embodiment, the stimulus unit 11 and the sensing unit 12 can be implemented as an integral source-meter unit 14. Advantageously, the stimulus unit 11 can comprise a digital-to-analog-converter (DAC). Such a DAC can even be co-integrated in a standard microcontroller (MCU) such as the STM32 series by ST Microelectronics. Correspondingly, the sensing unit 12 can comprise an analog-to-digital-converter (ADC) which may also be co-integrated in a standard microcontroller. Advantageously, the device 10 including the analysis unit 13 can thus be implemented using a microcontroller at low cost. However, the device may also be implemented as a distributed system. For example, data acquired by the sensing may be obtained (i.e. received or retrieved) by a remote processing unit, such as a smartphone running a corresponding app.

The sensing unit 12 provides the impedance signal 15 as an input to the analysis unit 13 which is adapted to identify an electrical double-layer based on the impedance signal acquired in response to the electrical stimulus provided by the stimulus unit 11 and to determine a state of psychophysiological responsiveness or non-responsiveness of the subject based the identified electrical double-layer. In particular, the analysis unit 13 can be adapted to determine a state of psychophysiological responsiveness or non-responsiveness of the subject based a transient behavior of said impedance signal 15 in response to the electrical the electrical stimulus provided by the stimulus unit 11. In this context, the analysis unit 13 can be adapted to provide a control signal 16 to the stimulus unit 11 for controlling the application of the electrical stimulus. In the alternative, the stimulus unit 11 may provide the analysis unit 13 with information about the provided electrical stimulus, such that the temporal relation between the provided electrical stimulus and the acquired impedance signal can be determined by the analysis unit 13.

Fig. 2 shows an exemplary implementation of a system 1 for assessing the psychophysiological responsiveness of the subject. For instance, the device can be implemented in form of a smart watch 40, skin conductance sensor wristband or activity tracker and the like. For example, the device may be implemented in the Philips discreet tension indicator (DTI-4) skin conductance sensor wristband. The device 40 comprises a main body 41 and a wristband 42. In the shown embodiment, the first electrode 21 and the second electrode 22, together indicated by reference numeral 20, are arranged on the bottom side of the housing 41 which, when worn by a subject, faces the skin of the subject. However, in an alternative embodiment, one or both electrodes may also be implemented on the wristband 42.

Referring again to Fig. 1, the skin of the subject 100 comprises the epidermis 102 as the upper portion and the dermis 103 as the lower portion of the skin 100. The topmost layer of the skin 100 as shown in Fig. 1 is the stratum corneum 101. For the case where the subject is in a state of psychophysiological responsiveness, the conductive path 25 within the skin 100 for the measurement of the skin impedance is exemplary shown by arrows. For simplification, the entire skin portion comprising the path 25, underneath the first and second electrodes 21, 22 can be thought of as being filled with a liquid medium comprising sweat as an electrolyte. Within the sweat, ion diffusion occurs when a voltage is applied across the first electrode 21 and the second electrode 22. This space can also be referred to as an interstitial space 103. In the sweat, ion diffusion occurs when an electrical stimulus such as a voltage step or plurality of voltage steps are applied over the first electrode 21 and the second electrode 22. In this context, the presence of a transient in the impedance signal 15 acquired by the sensing unit 12 signals the presence of a salty liquid, i.e. sweat, in the conductive path 25. The transients are caused by the (slow) diffusion of ion in the sweat, corresponding to changes in the electrical stimulus such as the applied voltage profile by lowering the space charge close to the electrodes. The space charge region is indicated by reference numeral 104. The space charge region indicates a region where an electrical double-layer forms. The electrical double-layer in turn provides a voltage barrier or blocking voltage that can be identified based on the impedance signal acquired in response to the electrical stimulus provided by the stimulus unit 11. As indicated in the lower section of Fig. 1, the sweat glands 106, when being filled with sweat as a conductive liquid, provide a conductive path towards the lower layers of the dermis 103.

On the other hand, if the subject is in a state of psychophysiological non-responsiveness, such a 'sweat path' is not available. In this case, if sweat is substantially absent, the conductive path is predominantly through the stratum corneum 101. Hence, ion diffusion does not happen in that case when an electrical stimulus is provided via the first and second electrodes 21, 22. Therefore, the response can be seen as an immediate change and not a slow change that gradually settles at a given value. Hence, the transient behavior, in particular due to the double-layer capacitance, can be evaluated. Further, if the sweat is substantially absent, the voltage barrier of the electrical double-layer, also referred to as ionic double-layer does not form. Hence, the presence or absence of such a voltage barrier can be evaluated.

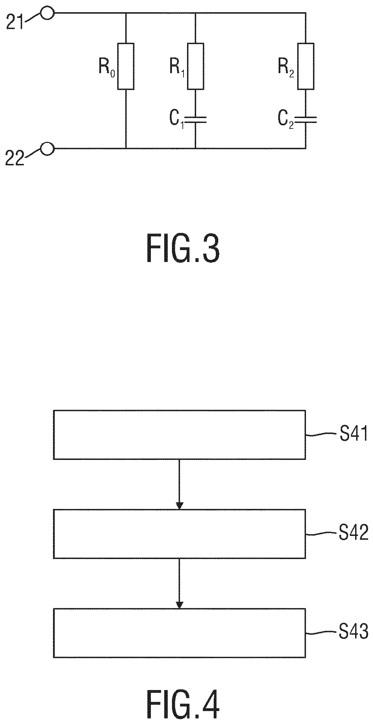

Fig. 3 shows a simplified equivalent electrical circuit of skin tissue and sweat glands. The equivalent circuit represents what is seen by the first electrode 21 and the second electrode 22 as the terminals. The skin may be modeled with an R-C circuit comprising a resistor R₀ in parallel with a first series connection of a resistor R₁ and a capacitor C₁, optionally in parallel with a second series connection of a resistor R₂ and a second capacitor C₂. This electrical network has also already been proposed by Comunetti et al. in "Characterisation of human skin conductance at acupuncture points", Experientia 51, 328-331, 1995, wherein it was shown that such an electrical network shows the same characteristic as human skin following a voltage step. The shown equivalent circuit is a good approximation if the subject is in a state of psychophysiological responsiveness wherein a capacitance is present due to ion diffusion of sweat in the sweat gland system. However, it has been found that if the subject is in a state of psychophysiological non-responsiveness, the behavior can be modeled by a series resistor only, hence, without the capacitive components by R₁ and C₁ in parallel with R₂ and C₂. For completeness, a resistor R₃ descriptive of a resistance of the stratum corneum is shown in Fig. 3. The stratum corneum can provide a large resistance, for example in the GΩ range. It is to be understood that while a capacitive component can be assumed to be present in any electrical circuit, the capacitance of the stratum corneum is orders of magnitude lower than a capacitive contribution from the sweat gland system, and has thus negligible effect on the switching behavior. In that case, the behavior may be dominated by the resistive component due to the stratum corneum. It should again be highlighted that even though the prior art already discloses that the skin may show a capacitive component in response to an applied voltage step, no analysis is performed to determine a state of psychophysiological responsiveness or non-responsiveness of the subject based on a transient behavior of an impedance signal or voltage barrier indicative of an electrical double-layer.

Further, the electrical double-layer provides a voltage barrier which can be modeled as a voltage source applied in series with the simplified parallel circuit shown in Fig. 3. A voltage drop across the voltage barrier can easily be identified, for example, by providing a first and a second voltage as electrical stimuli by the stimulus unit and evaluating an impedance signal in response thereto. For example, a sensing unit that evaluates a voltage drop across a reference resistor can be used as described above.

Fig. 4 shows an exemplary flow chart for assessing a psychophysiological responsiveness of a subject. In a first step S41, an electrical stimulus is provided via a first and a second electrode to a skin of the subject. In a second step S42, an impedance signal indicative of an impedance between the first and the second electrode in response to said electrical stimulus is acquired. In a third step S43, an electrical double-layer is identified based on the impedance signal acquired in response to the electrical stimulus and a state of psychophysiological responsiveness or non-responsiveness of the subject is determined based the identified electrical double-layer.

Fig. 5A to Fig. 5D show exemplary graphs of provided electrical stimuli and acquired impedance signals. The device for providing these electrical stimuli and acquiring the corresponding impedance signals can be a wearable device as exemplarily shown in Fig. 2. For performing a skin conductance measurement, a constant measuring voltage of, for example, 1.048 V can be used. However, when assessing a psychophysiological responsiveness of the subject, the electronic design can be adapted to provide a voltage profile via the first and the second electrode 21, 22 wherein the voltage can be varied, for example lowered in a stepwise manner as indicated by the curve denoted by reference numeral 51.

In Fig. 5A to Fig. 5D, the horizontal axis denotes time in seconds. The dashed curve 51 denotes the electrical stimulus provided by the stimulus unit via the first and the second electrode to a skin 100 of the subject. The solid lines 52 indicate a skin conductance value as the impedance signal indicative of the impedance between the first and the second electrode in response to said electrical stimulus 51. The vertical axis on the left side denotes a skin conductance value (SC) in arbitrary units corresponding to curve 52. The vertical axis on the right side denotes a voltage indicator of a voltage U as a percentage to a reference voltage in percent [% of Vref] corresponding to curve 51.

Compared to a conventional device for measuring a skin conductance, the device can simply be adapted by replacing the reference voltage module for skin conductance measurement with a programmable voltage module, such as a digital-to-analog-converter (DAC). In the shown embodiment, the skin conductance as a special case of the impedance is measured at a sampling rate of about 160 Hz when in a sequence of 10 steps the voltage is lowered from about 1 V down to 0.1 V. The results of such measurements for four human test subjects are shown in Fig. 5A to Fig. 5D. The impedance signals can for example be measured at a sampling rate of 100 Hz or more, preferably 160Hz. Referring to Fig. 5A and Fig. 5B, the skin conductance traces for persons 1 and 2 on the one hand show an almost immediate stepwise response to voltage changes, as indicated by reference numeral 53. A transient behavior is thus virtually absent. These persons were in a state of psychophysiological non-responsiveness. Artifacts that may occur when switching to a different voltage level may be neglected in the analysis. On the other hand, the skin conductance traces for persons 3 and 4, the settling of the acquired skin conductance value takes up to 10 s. These persons were in a state of psychophysiological responsiveness. In contrast to the stepwise response as indicated by reference numeral 53, the skin conductance for persons 3 and 4 shows a transient behavior as indicated by reference numeral 54.

An exemplary suitable algorithm to detect the presence of the transient behavior after voltage changes may advantageously use the first derivative of the impedance signal 52. In the absence of transient behavior as shown in Fig. 5A and Fig. 5B, the first derivative will be a small and stable value. However in contrast, in the case of a transient behavior as shown in Fig. 5C and Fig. 5D, the first derivative can be expected to go to a large positive or negative value shortly after the corresponding, here declining, voltage step, and gradually over a couple of seconds declines to a low value.

In addition or as an alternative to evaluating a transient behavior for identifying the electrical double-layer, it is also possible to evaluate the voltage barrier indicative of said electrical double-layer based on the impedance signal by the skin acquired in response to the electrical stimulus.

For acquisition of the impedance signal indicative of the skin conductance between the first and the second electrode, a circuit as described in detail in WO 2016/050 551 A1, the content of which is incorporated herein by reference, can be used. Since, in the embodiment described with reference to Fig. 5A to Fig. 5D, the stimulus unit provides a voltage profile, as indicated by dashed curve 51, wherein the voltage is changed, a correction can be applied to account for the different voltage levels by G skin corrected = G skin measured x Vref/Vstimulus. For a non responsive state this leads to the increase in skin conductance visible in figures 5A and 5B. For a responsive state this leads to a decrease in skin conductance as visible in Figures 5C and 5D. Hence, a change in an absolute value of the determined (corrected) can be evaluated to identify the electrical double-layer. An advantage of this embodiment is that a slow measurement can be performed. Hence, slower and potentially less expensive components may be used. Furthermore, the power consumption can be reduced if a slow sampling rate is sufficient. This is particularly advantageous in wearable devices.

Optionally, the comparison of such a corrected skin conductance can be made between an average of, for example, the last 4 to 160, preferably the last 10 data points if the impedance signal is measured at a sampling rate of 160Hz, before and after a voltage step or switch. In the case where no difference can be found, the voltage step can be doubled and the measurement repeated. For example, a sensitivity of an ADC (10bit ... 16bit) in the sensing unit can vary. Hence, in order to yield a detectable effect, the voltage steps provided by the stimulus unit can be doubled in the absence of a measured effect.

In an embodiment, the analysis can optionally also be combined with an absolute measurement wherein the analysis unit is configured to evaluate whether a skin conductance level is below a predetermined threshold, for example below 1 micro Siemens, otherwise the subject is considered to be in a state of psychophysiological responsiveness

In a wearable skin conductance sensor, it can be especially important to determine whether a period without skin conductance responses signifies a period of relaxation. For example, the voltage step or generally speaking the electrical stimulus used for assessing the psychophysiological responsiveness of the subject can be applied in a predetermined interval of for example 10s or 7s during such a skin conductance response (SCR) free period. Hence, a device for skin conductance measurement can be adapted to assessing a psychophysiological state of the subject after a predetermined period without a skin conductance response. It can thus be verified whether the subject actually is in a period of relaxation or determined whether the subject is in a state of psychophysiological non-responsiveness. A typical skin conductance measurement can take about 7 seconds, from start to end, so a slightly shorter interval can assure that no responses are missed.

It should be noted that it is not mandatory to apply a voltage profile as shown in Fig. 5A to Fig. 5C. A single voltage step, for example a declining voltage step of 20 to 50 % can also be sufficient. For evaluating a transient behavior, the applied voltage can, for example, be lowered in less than 10 ms, preferably in less than 5 ms After such a step a standard measurement voltage for skin conductance measurement can be re-established. A further example of a voltage profile comprises reversing or commutating an applied voltage. An advantage of this embodiment lies in the cost effective implementation, for example, by means of switches for commutating the applied voltage.

Referring again to Fig. 1, an optional interface or output unit 30 can be provided. For example, the interface can be a data interface which provides data indicative of the state of psychophysiological responsiveness or non-responsiveness of the subject. Such information can also be provided to the user on a human-machine-interface (HMI) for example on the screen of a smartphone. Should a state of non-responsiveness be detected, an advice of how to mitigate this can also be given. For example, simply moistening the area under the electrode with tap water may create a liquid medium in which the transient behavior can be expected to re-appear as well as the psychophysiological responsiveness. It should be noted that executing assessment of the psychophysiological responsiveness of the subject can again be used to verify the effectiveness of these measures.

In conclusion, an advantageous approach for assessing a psychophysiological responsiveness of a subject has been presented that enables the determination a state of psychophysiological responsiveness or non-responsiveness of a subject. The proposed solution can be added to skin conductance measurement devices with limited cost and effort. It should be understood that the device for assessing a psychophysiological responsiveness of the subject can also advantageously be applied in fields where a placement of electrodes - in generally the contact of an electrode to the skin of the subject - is to be verified. Exemplary applications include electrocardiogram (ECG), electromyogram (EMG) or electroencephalogram (EEG) applications. Hence, the rather complex procedure of applying wet electrodes or an electrode gel underneath electrodes to ensure a good electrode skin contact can be simplified. A state of psychophysiological responsiveness can thus be equivalent to a good electrode skin contact in particular an electrode-skin contact comprising a capacitive contribution from the skin of the subject. Correct contact to the skin of the subject can thus be verified. In passive methods such as EEG and EEG, an additional stimulus unit for providing an electrical stimulus to the skin of the subject would be required. It should, however, be appreciated that such it is sufficient to temporarily apply a stimulus unit when preparing an actual measurement.

While the invention has been illustrated and described in detail in the drawings and foregoing description, such illustration and description are to be considered illustrative or exemplary and not restrictive. The invention is defined by the appended claims. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. Device (10) for assessing a psychophysiological responsiveness of a subject, the device comprising:
- a stimulus unit (11) for providing an electrical stimulus via a first and a second electrode (21, 22) to a skin (100) of the subject;
- a sensing unit (12) for acquiring an impedance signal (14) indicative of an impedance between the first and the second electrode in response to said electrical stimulus; and
- an analysis unit (13) adapted to identify an electrical double-layer based on the impedance signal acquired in response to the electrical stimulus and to determine a state of psychophysiological responsiveness or non-responsiveness of the subject based on the identified electrical double-layer,
wherein the analysis unit is configured to identify the electrical double-layer based on a transient behavior of said impedance signal,
wherein the analysis unit (13) is adapted to determine the state of psychophysiological responsiveness of the subject based on a transient behavior of a time scale of 1 ms to 5 s,
wherein the stimulus unit (11) comprises a voltage source for applying a voltage across the first and the second electrode (21, 22) and/or a current source for applying a current to the first and the second electrode and
wherein the stimulus unit (11) is adapted to provide an electrical stimulus comprising a step function from a first voltage to a second voltage or from a first current to a second current.

2. Device according to claim 1, wherein the analysis unit (13) adapted to discriminate between a state of psychophysiological responsiveness and psychophysiological non-responsiveness, wherein an absence of an electrical double-layer is indicative of a state of psychophysiological non-responsiveness of the subject.

3. Device according to claim 1, wherein the analysis unit (13) adapted to discriminate between a state of psychophysiological responsiveness and psychophysiological non-responsiveness, wherein a presence of an electrical double-layer is indicative of a state of psychophysiological responsiveness of the subject.

4. Device according to claim 1, wherein the analysis unit is configured to identify the electrical double-layer by evaluating a voltage barrier indicative of said electrical double-layer based on the impedance signal acquired in response to the electrical stimulus.

5. Device according to claim 1, wherein the analysis unit (13) is further adapted to determine the state of psychophysiological responsiveness of the subject based on a transient behavior of a time scale between 10 ms and 2 s, preferably between 20 ms and 500 ms.

6. Device according to claim 1, wherein the stimulus unit (11) is adapted to provide a voltage and/or current profile.

7. Device according to claim 1, wherein the stimulus unit (11) is adapted to provide an AC electrical excitation.

8. Device according to claim 1, wherein the sensing unit (12) is adapted to acquire a time-variant current and/or voltage between the first and the second electrode.

9. Device according to claim 1, further comprising an output unit (30) for providing information associated with the identified state of psychophysiological responsiveness or non-responsiveness to the subject, the information comprising providing advice how to overcome a state of psychophysiological non-responsiveness.

10. System (1) for assessing a psychophysiological responsiveness of a subject, the system comprising:
- a first electrode (21) and a second electrode (22) for application to a skin (100) of the subject; and
- the device (10) for assessing the psychophysiological responsiveness of the subject according to claim 1.

## Patentansprüche

1. Vorrichtung (10) zum Bewerten der psychophysiologischen Reagibilität eines Subjekts, die Vorrichtung umfassend:
- eine Stimulationseinheit (11) zum Bereitstellen elektrischen Reizes über eine erste und eine zweite Elektrode (21, 22) an eine Haut (100) des Subjekts;
- eine Sensoreinheit (12) zum Erfassen eines Impedanzsignals (14), das indikativ für eine Impedanz zwischen der ersten und der zweiten Elektrode als Reaktion auf den elektrischen Reiz ist; und
- eine Analyseeinheit (13), angepasst ist, um eine elektrische Doppelschicht basierend auf dem als Reaktion auf den elektrischen Reiz erfassten Impedanzsignals zu identifizieren und einen Zustand der psychophysiologischen Reagibilität oder Nicht-Reagibilität des Subjekts basierend auf der identifizierten elektrischen Doppelschicht zu bestimmen, wobei die Analyseeinheit konfiguriert ist, um die elektrische Doppelschicht basierend auf einem transienten Verhalten des Impedanzsignals zu identifizieren, wobei die Analyseeinheit (13) angepasst ist, um den Zustand der psychophysiologischen Reagibilität des Subjekts basierend auf einem transienten Verhalten auf einer Zeitskala von 1 ms bis 5 s zu bestimmen, wobei die Stimulationseinheit (11) eine Spannungsquelle zum Anlegen einer Spannung an die erste und die zweite Elektrode (21, 22) und/oder eine Stromquelle zum Anlegen eines Stroms an die erste und die zweite Elektrode umfasst und wobei die Stimulationseinheit (11) angepasst ist, um einen elektrischen Stimulus bereitzustellen, der eine Schrittfunktion von einer ersten Spannung zu einer zweiten Spannung oder von einem ersten Strom zu einem zweiten Strom umfasst.

2. Vorrichtung nach Anspruch 1, wobei die Analyseeinheit (13) angepasst ist, um zwischen einem Zustand der psychophysiologischen Reagibilität und der psychophysiologischen Nicht-Reagibilität zu unterscheiden, wobei ein Fehlen einer elektrischen Doppelschicht indikativ für einen Zustand einer psychophysiologischen Nicht-Reagibilität des Subjekts ist.

3. Vorrichtung nach Anspruch 1, wobei die Analyseeinheit (13) angepasst ist, um zwischen einem Zustand psychophysiologische Reagibilität und psychophysiologischer Nicht-Reagibilität zu unterscheiden, wobei das Vorhandensein einer elektrischen Doppelschicht indikativ für einen Zustand psychophysiologischer Reagibilität des Subjekts ist.

4. Vorrichtung nach Anspruch 1, wobei die Analyseeinheit konfiguriert ist, um die elektrische Doppelschicht zu identifizieren, indem sie eine Spannungsbarriere auswertet, die indikativ für die elektrische Doppelschicht basierend auf dem Impedanzsignals ist, das als Reaktion auf den elektrischen Stimulus erfasst wird.

5. Vorrichtung nach Anspruch 1, wobei die Analyseeinheit (13) ferner angepasst ist, um den Zustand psychophysiologischer Reagibilität des Subjekts basierend auf einem transienten Verhalten auf einer Zeitskala zwischen 10 ms und 2 s, vorzugsweise zwischen 20 ms und 500 ms, zu bestimmen.

6. Vorrichtung nach Anspruch 1, wobei die Stimulationseinheit (11) angepasst ist, um ein Spannungs- und/oder Stromprofil bereitzustellen.

7. Vorrichtung nach Anspruch 1, wobei die Stimulationseinheit (11) angepasst ist, um eine elektrische AC-Wechselstromerregung bereitzustellen.

8. Vorrichtung nach Anspruch 1, wobei die Erfassungseinheit (12) angepasst ist, um einen zeitlich veränderlichen Strom und/oder eine zeitlich veränderliche Spannung zwischen der ersten und der zweiten Elektrode zu erfassen.

9. Vorrichtung nach Anspruch 1, ferner umfassend eine Ausgabeeinheit (30) zum Bereitstellen von Informationen, die mit dem identifizierten Zustand psychophysiologischer Reagibilität oder Nicht-Reagibilität für das Subjekt assoziiert sind, wobei die Informationen ein Bereitstellen von Ratschlägen umfassen, wie ein Zustand psychophysiologischer Nicht-Reagibilität überwunden werden kann.

10. System (1) zum Bewerten einer psychophysiologischen Reagibilität eines Subjekts, das System umfassend:
- eine erste Elektrode (21) und eine zweite Elektrode (22) zum Aufbringen auf eine Haut (100) des Subjekts; und
- die Vorrichtung (10) zum Bewerten der psychophysiologischen Reagibilität des Subjekts nach Anspruch 1.

## Revendications

1. Dispositif (10) d'évaluation d'une réactivité psychophysiologique d'un sujet, le dispositif comprenant:
- une unité de stimulus (11) pour fournir un stimulus électrique via une première et une seconde électrode (21, 22) à une peau (100) du sujet;
- une unité de détection (12) pour acquérir un signal d'impédance (14) indicatif d'une impédance entre la première et la seconde électrode en réponse audit stimulus électrique; et
- une unité d'analyse (13) adaptée pour identifier une double couche électrique sur la base du signal d'impédance acquis en réponse au stimulus électrique et pour déterminer un état de réactivité ou de non-réactivité psychophysiologique du sujet sur la base de la double couche électrique identifiée, dans lequel l'unité d'analyse est configurée pour identifier la double couche électrique sur la base d'un comportement transitoire dudit signal d'impédance, dans lequel l'unité d'analyse (13) est adaptée pour déterminer l'état de réactivité psychophysiologique du sujet sur la base d'un comportement transitoire d'une échelle de temps de 1 ms à 5 s, dans lequel l'unité de stimulation (11) comprend une source de tension pour appliquer une tension aux bornes de la première et de la seconde électrodes (21, 22) et/ou une source de courant pour appliquer un courant à la première et à la seconde électrodes et dans lequel l'unité de stimulus (11) est adaptée pour fournir un stimulus électrique comprenant une fonction échelon d'une première tension à une seconde tension ou d'un premier courant à un second courant.

2. Dispositif selon la revendication 1, dans lequel l'unité d'analyse (13) adaptée pour discriminer entre un état de réactivité psychophysiologique et d'apathie psychophysiologique, dans lequel une absence de double couche électrique est révélatrice d'un état d'apathie psychophysiologique de l'objet.

3. Dispositif selon la revendication 1, dans lequel l'unité d'analyse (13) est adaptée pour discriminer entre un état de réactivité psychophysiologique et une non-réactivité psychophysiologique, dans lequel une présence d'une double couche électrique est indicative d'un état de réactivité psychophysiologique du sujet.

4. Dispositif selon l'une quelconque des revendications 1 précédentes, dans lequel l'unité d'analyse est configurée pour identifier la double couche électrique en évaluant une barrière de tension indicative de ladite double couche électrique à partir du signal d'impédance acquis en réponse au stimulus électrique.

5. Dispositif selon la revendication 1, dans lequel l'unité d'analyse (13) est en outre adaptée pour déterminer l'état de réactivité psychophysiologique du sujet en fonction d'un comportement transitoire d'une échelle de temps comprise entre 10 ms et 2 s, de préférence entre 20 ms et 500 ms.

6. Dispositif selon l'une quelconque des revendications 1 précédentes, dans lequel l'unité de stimulation (11) est adaptée pour fournir un profil de tension et/ou de courant.

7. Dispositif selon la revendication 1, dans lequel l'unité de stimulation (11) est adaptée pour fournir une AC excitation électrique alternative.

8. Dispositif selon l'une des revendications 1, dans lequel l'unité de détection (12) est adaptée pour acquérir un courant et/ou une tension variant dans le temps entre la première et la deuxième électrode.

9. Dispositif selon la revendication 1, comprenant en outre une unité de sortie (30) pour fournir des informations associées à l'état identifié de réactivité ou de non-réactivité psychophysiologique au sujet, les informations comprenant la fourniture de conseils sur la manière de surmonter un état de non-réactivité psychophysiologique.

10. Système (1) pour évaluer une réactivité psychophysiologique d'un sujet, le système comprenant:
- une première électrode (21) et une seconde électrode (22) pour application sur une peau (100) du sujet; et
- le dispositif (10) d'évaluation de la réactivité psychophysiologique du sujet selon la revendication 1.
